# EUROPEAN PATENT SPECIFICATION

(11) **EP 1 675 891 B1**
(45) Date of publication and mention of the grant of the patent: **30.05.2007**
(21) Application number: 04793897.2
(22) Date of filing: 13.10.2004
(51) Int. Cl.: C08J 3/28, C08L 5/06, A61K 9/00, C08J 5/18

(54) **PECTIN FILMS PREPARED FROM GAMMA IRRADIATED PECTIN**
AUS MIT GAMMA-STRAHLEN BESTRAHLTEM PEKTIN HERGESTELLTE PEKTINFILME
FILMS DE PECTINE PREPARES A PARTIR DE PECTINE IRRADIEE AUX RAYONS GAMMA

(30) Priority: 14.10.2003 US 510518 P; 28.09.2004 US 950631
(43) Date of publication of application: 05.07.2006
(73) Proprietor: CP Kelco, U.S., Inc., Atlanta, GA 30339 (US)
(72) Inventor: CLARK, Ross, San Diego, CA 92131-3630 (US)
(74) Representative: Jacob, Reuben Ellis
(86) International application number: PCT/US2004/030919
(87) International publication number: WO 2005/037895

(56) References cited:
- US-A1- 2003 027 883
- PATENT ABSTRACTS OF JAPAN vol. 1998, no. 09, 31 July 1998 (1998-07-31) & JP 10 101704 A (JAPAN ATOM ENERGY RES INST), 21 April 1998 (1998-04-21)

## Description

### FIELD OF THE INVENTION

The invention relates to pectin films having rapid dissolution rates.

### BACKGROUND OF THE INVENTION

Films formed from water-soluble polymers can be used to administer various pharmaceutical compounds, including drugs and orally active agents such as breath fresheners. Examples of such polymers are disclosed in, for example, U.S. Patents 6,419,903; 6,284,264; 6,177,096; and 5,948,430.

Polymer solutions are cast onto a suitable surface and then dried to form a film. More specifically, the film is formed by mixing a high concentration polymer solution or "dope" and then adding the active agents along with plasticizing agents and other ingredients. A thin film or shape is cast and the final product results from drying this film or shape.

The use of pectin and many other water-soluble polymers are well known, see for example U.S. 6,197,331. A pectin solution can be formed from many types of pectin including high methoxyl, low methoxyl, and low methoxyl-amidated type pectins. Usually the pectin solution is deposited at a high concentration of pectin (from 3 wt% to as much as 15 wt% pectin solids). The pectin is cast on a suitable metal or polymeric surface to deposit a smooth film, without voids, which can be removed from the surface with minimal damage. Drying can be carried out by any suitable technique such as room temperature air-drying, heated belt drying, and refractive window drying on a Mylar or similar plastic belt.

In another process, the quality of pectin films is improved by reducing the molecular weight of the pectin. For example, the molecular weight may be reduced by adjusting the pH to near 7. This causes beta elimination and a loss of molecular weight. This process was commercially introduced by Aquafilm in Tampa, FL. It is also possible to use enzymes, mechanical shear and oxidizing agents such as H₂O₂ to reduce the degree of polymerization of the pectin.

The reduction of molecular weight Mw is important for a number of reasons. It allows the solution that will be cast as a film to be prepared at a higher polymer loading meaning that less water will have to be removed during drying. The film dissolves more quickly when exposed to water. The film usually requires a lower level of plasticizing agent such as glycerin or sorbitol. The films made with lower molecular weight are stiffer.

### BRIEF SUMMARY OF THE INVENTION

It was discovered that irradiating the pectin with gamma radiation will lead to pectin with reduced molecular weight and improved film properties. Films produced by pectin treated with gamma radiation are also clearer than the previously made films that used pH treatment to reduce the molecular weight.

The invention is directed to a process for making a rapidly dissolving (quick-release) pectin film, comprising the steps of: treating pectin with gamma radiation, forming a pectin solution with the treated pectin; and forming a film from the pectin solution. Alternatively, the process comprises treating a pectin solution with gamma radiation; and forming a film from the pectin solution. These processes of producing the rapidly dissolving pectin film are easy and uncomplicated.

A pectin solution produced from pectin treated with gamma radiation is suitable for film casting and provides quicker dissolution than solutions prepared from untreated pectin. This in turn allows an increased dissolution rate and also provides the pectin with better flavor properties and a less gummy mouthfeel when used for oral applications.

The invention is directed to a pectin film composition that is rapidly dissolvable in water, the composition comprising a high or low methyl ester pectin treated with gamma radiation and at least one active agent.

The rapidly dissolvable pectin films are useful for, for example, drug delivery and breath films. The pectin films formed in accordance with the instant invention have an adjustable dissolution rate, have good hydration to make casting "dope," and do not have off odors or flavors. In addition, the films are consistently strong and flexible, do not mask flavors or perfumes, and are compatible with active agents.

The invention is further directed to an orally consumable film composition for delivering at least one active agent to an oral cavity wherein the film is rapidly dissolvable in the oral cavity, the composition comprising a high or low methyl ester pectin treated with gamma radiation. The film may further comprise an active agent or a flavoring aid.

The invention is further directed to rapidly dissolvable films that are applied to skin or hair. The skin or hair is generally moistened with water and the film applied, and optionally, more water is applied. Alternatively, the film may be applied and then moistened with water. The film is rapidly dissolvable in water and comprises a high or low methyl ester pectin treated with gamma radiation and at least one active agent such as a skin softening agent or hair color.

The invention is further directed to films that are applied to skin to aid in healing of wounds. The film comprises a high or low methyl ester pectin treated with gamma radiation.

BRIEF DESCRIPTION OF THE DRAWINGS

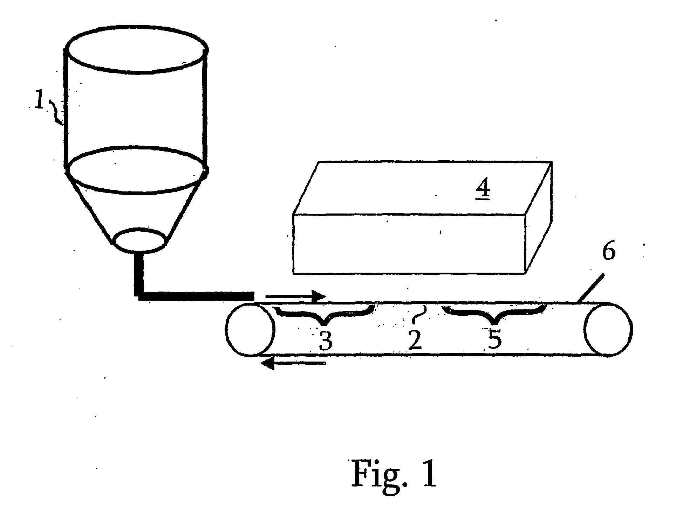

Figure 1 depicts one embodiment for preparing the pectin films.

### DETAILED DESCRIPTION OF THE INVENTION

The present invention is directed to a film comprising pectin treated with gamma radiation. The film rapidly dissolves in water. The film preferably further comprises one or more active ingredients and, when the film dissolves, the film releases the active agent and/or flavoring aid. The agent may be a therapeutic agent, a pharmaceutical agent, and/or an orally active agent. The film is suitable for oral uses as it rapidly dissolves in the mouth.

It was discovered that when the pectin is treated with gamma radiation, a film prepared with the pectin dissolves in aqueous media more rapidly. This reduces the need for additional plasticizer, such as polyethylene glycol (PEG) and glycerin, which can make films tacky and stick together under high humidity conditions.

Pectin is available in USP grades and has few regulatory barriers around the world. Some competitive products such as gelatin, modified starch, and synthetic polymers have more regulatory barriers.

The inventive process thus modifies the pectin to allow the film to dissolve very quickly and completely. A film that dissolves more quickly will release the active agent contained in the film more rapidly. This is desired, for example, in a breath film for a flavor with an initial high impact.

Films prepared from using pectin treated with gamma radiation dissolve much more quickly in the mouth and release the flavor much more readily than do films prepared from untreated pectin. Rapid dissolution and release of the active ingredient is especially important in applications such as breath films. For instance, films prepared from pectin treated with gamma radiation have a greater intensity of flavor than prior art films and the flavor remains at high levels during storage.

The film also has instant wettability, which allows the film to soften immediately after application to moisture or the mucosal tissue. In the case of orally delivered films, this prevents the patient from experiencing any prolonged adverse feeling in the mouth. In the case of skin application, this property could also assist in the quick delivery of medications, for example, one that was directed at healing the skin or dealing with psoriasis.

The film also has a tensile strength suitable for normal coating, cutting, slitting, and packaging operations.

The pectin film may be formed from many types of pectin including high methoxyl, low methoxyl, and low methyl ester amidated type pectins. Preferably, high methoxyl pectins are used. The galacturonic acid residues in pectin are partly esterified and present as the methyl ester. The degree of esterification is defined as the percentage of carboxyl groups esterified. Pectin with a degree of esterification ("DE") above 50% is named high methyl ester ("HM") pectin or high ester pectin and one with a DE lower than 50% is named low methyl ester ("LM") pectin or low ester pectin. Most pectin found in fruits and vegetables are HM pectins. One suitable HM pectin is D Slow Set (available from CP Kelco ApS).

The pectin is treated with gamma radiation. Gamma radiation can be generated from any suitable source, typically from an electron beam or a cobalt source. The electron beam source is well controlled but penetrates less than the cobalt-produced gamma. While cobalt gamma and electron beam are not directly interchangeable, they do have a similar effect on the pectin when used at the same dosage.

The radiation dosage can range from about 10 to about 50 KGy, preferably about 10 to about 30 KGy, more preferably about 10 to about 20 KGy, and most preferably about 15 KGy. Generally, more than 25 to 30 KGy reduces the molecular weight to the extent that a coherent film may not be formed. Instead the film may break into pieces similar to a sugar coating. Amounts less than about 10 KGy generally provide a slower dissolving film. Treatment at about 15 KGy provides a product similar to a product made with pH neutralization to pH 6.5 and is generally considered optimal.

The electron beam exposure to the pectin is fairly short in duration while the cobalt source is exposed to the product for a period of some minutes, depending upon the intensity of the cobalt source in use. Dosimeters are used on the outside of the package to determine the level of radiation applied. Penetration is assumed to be 10 cm or less for the dry powdered pectin.

It is possible to irradiate a solution as well as the dry product. In most cases, treatment of the dry product is preferred since there is less weight of material to be treated and it is easier to prepare pectin solutions once they have been treated. After the pectin is treated with the gamma radiation, it may be combined with a plasticizer or any other suitable ingredients, such as flavoring aids, colorants, pharmaceuticals, therapeutic agents, and the like and then formed into a film. Penetration of the radiation can be less for the solution than the dry product.

Suitable amounts of plasticizing agents can be added to enhance the extensibility of the film and thus increase its strength. Plasticizing agents are normally used at a ratio , of about 1:10 to 2:1 based upon the amount of pectin present. That is, the plasticizing material can be as little as 1/10 the weight of the pectin or as much as 2 times the pectin weight. In most cases, the preferred ratio is 1:4 to 1:1.5. This will give a film that is still flexible but not too sticky and tacky. Less plasticizing agent makes the film stiff and brittle. Higher levels cause the film to soften and become flexible as well as sticky if too much is used. (Although sticky films can be used for adhesives in certain cases.) Most lower molecular weight non-ionic soluble materials can be used as plasticizing agents. Preferred materials are glycerin, sorbitol, propylene glycol, poly-ethylene glycol and similar compounds.

The films produced from gamma radiation treated pectin can be used to administer various pharmaceutical compounds, such as therapeutic agents, drugs, and breath fresheners.

Active breath freshening agents can be incorporated into the film composition of the present invention to form the breath freshening strips of the present invention. The active ingredients include zinc gluconate, zinc citrate and/or alpha ionone. These agents function in masking mouth odor and reducing volatile odor causing bacterial sulfur compounds. These agents may be incorporated in the film composition of the present invention at a concentration of about 0.1 to about 2.0% by weight and preferably about 0.15 to about 0.5% by weight.

Active ingredients for relief of sore throats may also be included such as menthol and benzocaine.

The films may also be used to apply to wounds such as shaving cuts. In this case, ingredients, such as emollients, can be incorporated into the film composition.

The films may also be used for face masks, makeup, acne-treatment, and sun block for application to the skin. The films may be used to apply color to hair, in particular when applying highlights. When used for the skin, abrasives such as silica may be added to the film solution for cleansing products and skin masks. The films may be used for specialty make up containing, for example, glitter flakes or particles.

The films may also be used as fun skin films for children. Such films may be provided in a variety of colors or mixture of colors and may be used for cleansing.

The film can further comprise, for example, water, additional film forming agents, flavoring agents, antimalodor agents, surfactants, emulsifying agents, coloring agents, sweeteners and fragrances.

Flavoring aids include those known to the skilled artisan, such as natural and artificial flavors. These flavorings may be chosen from synthetic flavor oils and flavoring aromatics, and/or oils, oleo resins and extracts derived from plants, leaves, flowers, fruits and so forth, and combinations thereof. Representative flavor oils include: spearmint oil, cinnamon oil, peppermint oil, clove oil, bay oil, thyme oil, cedar leaf oil, oil of nutmeg, oil of sage, and oil of bitter almonds. These flavor agents can be used individually or in admixture. Commonly used flavors include mints such as peppermint, artificial vanilla, cinnamon derivatives, and various fruit flavors, whether employed individually or in admixture. Generally, any flavoring or food additive, such as those described in Chemicals Used in Food Processing, publication 1274 by the National Academy of Sciences, pages 63-258, may be used. The amount of flavoring agent employed is normally a matter of preference subject to such factors as flavor type, individual flavor, and strength desired. Generally the flavoring is incorporated in the film of the present invention in an amount ranging from about 2.0 to about 20% by weight and preferably about 5 to about 10% by weight.

Sweeteners useful in the practice of the present invention include both natural and artificial sweeteners. Suitable sweetener include water soluble sweetening agents such as monosaccharides, disaccharides and polysaccharides such as xylose, ribose, glucose (dextrose), mannose, glatose, fructose (levulose), sucrose (sugar), maltose, water soluble artificial sweeteners such as the soluble saccharin salts, i.e., sodium or calcium saccharin salts, cyclamate salts dipeptide based sweeteners, such a L-aspartic acid derived sweeteners, such as L-aspartyl-L-phenylalaine methyl ester (aspartame) or other sweeteners such as Sucralose.

In general, the effective amount of sweetener is utilized to provide the level of sweetness desired for a particular composition, will vary with the sweetener selected. This amount will normally be about 0.01% to about 2% by weight of the composition.

The compositions of the present invention can also contain coloring agents or colorants. The coloring agents are used in amounts effective to produce the desired color and include natural food colors and dyes suitable for food, drug and cosmetic applications. These colorants are known as FD&C dyes and lakes. The materials acceptable for the foregoing spectrum of use are preferably water-soluble, and include FD&C Blue No.2, which is the disodium salt of 5,5-indigotindisulfonic acid. Similarly, the dye known as Green No.3 comprises a 15 triphenylmethane dye and is the monosodium salt of 4-[4-N-ethyl-p-sulfobenzylamino) diphenyl-methylene ]-[1-N-ethy 1-N-sulfonium benzyl)-2,5-cyclo-hexadienimine]. A full recitation of all FD&C and D&C dyes and their corresponding chemical structures may be found in the Kirk-Othrner Encyclopedia of Chemical Technology, Volume 5, Pages 857-884, which text is accordingly incorporated herein by reference.

Figure 1 shows one embodiment of the film making operation. First the polymer solution or dope is prepared and mixed well to obtain a high polymer loading. The mixing tank (1) may be temperature controlled and a second surge tank may be plumbed into the system to allow for continuous operation.

Following the mixing step, the solution is cast (evenly distributed) on a releasable carrier such as a belt (2). A manifold, blades, nozzles, and/or other suitable equipment may be used to cast the polymer. The carrier material must have a surface tension, which allows the film solution to spread evenly across the intended carrier width without soaking to form a destructive bond between the film and carrier substrates. Examples of suitable carrier materials include glass, PET plastic, stainless steel, Teflon and polyethylene-impregnated paper.

It is desirable to have the casting solution or dope at as high a polymer loading as possible. This leaves less water to be removed in the drying process and leads to a film that will curl less and dry more quickly. Quick drying will also preserve volatile ingredients like flavors. Pectin may be prepared to concentrations of 10% or even more when properly treated. Depending upon the system used to cast the film onto the belt, the ideal viscosity of the dope can range from 5,000 cP to over 80,000 cP. Preferably from 50,0000 to 80,000 cP, more preferably from 65,000 to 75,000 cP. The dope may also be heated before putting in on the belt, which reduces viscosity, decreases drying times and eases material handling.

The cast film should be of even thickness and quite a bit thicker than the final film. As it dries, the film will not contract so much from side to side as it will become thinner in cross section. For example, a film with a final thickness of 0.0015 inches (1.5 mils) and a solids loading of the dope of 10%, should have an initial film thickness of about 0.012 inches (12 mils).

The composition is cast on the releasable carrier and dried. Drying of the film may be carried out at high temperature using a drying oven, drying terminal, vacuum drier, or any other suitable drying equipment, which does not adversely affect the ingredients of which the film is composed.

In the figure, the cast film on the belt passes over a heating section (3). Heat can be applied in any suitable manner such as steam or infrared radiation. The cast film and belt can be heated from below or above or even both sides. Typically, a hood (4) or other arrangement is used over the belt to remove the moisture and control the environment while drying. Preferably, the drying will be monitored such as with a reflected infrared instrument. Excessive heat should be avoided since it causes the film to curl after storage. Water activity of the product when dried should be about 0.3 to 0.5 (30% to 50% RH). The cast film on the belt may then pass over a cooling section (5). Cooling may be used after the heating step to prepare the film for removal from the belt.

The film is then removed from the carrier in any suitable manner. In the figure, the film is removed from the belt with a scraper blade (6). Clean removal from the belt is essential for a smooth running operation. To aid in removal, a surfactant may be applied to the belt before drying starts. In addition, because the pectin has a low viscosity and high polymer loading, removal is relatively easy. Excessive amounts of plasticizing agent should be avoided as it will cause the film to stick to the belt.

Once removed, the film may be cut into strips and wound onto rolls. In some cases, this intermediate processing, often called "conversion" by the plastic or film industry, can be done with the backing or carrier sheet still bonded to the film. In these cases, the backing is tripped from the film immediately before being placed in the package. The film may also be segmented into dosage units by die-cutting or slitting-and-die-cutting. The segmented film has a strip width of generally about 15 to about 30 millimeter and a length of about 20 to about 50 millimeters in length. The film has a thickness ranging from about 10 to about 200 micrometers, and preferably about 25 to 75 micrometers.

If used orally, the film is preferably shaped and sized for placement in the mouth. The film is flexible and adheres to a surface in the mouth, usually the roof of the mouth or the tongue, and quickly dissolves, typically in less than two minutes, preferably from 15 seconds to one minute. Dissolution speed can be controlled thickness of the film.

The films produced from gamma radiation treated pectin in accordance with the invention has a dissolution rate of at least 10 %, preferably at least 25%, more preferably at least 35%, over untreated pectin. Typically improvements range from 25 to 50% improvement.

Example 1

Ten 2 Kg samples of CP Kelco high methyl pectin, D Slow Set Z were placed in individual plastic zip lock bags and were treated with gamma radiation from an electron beam source. The dose level was set to 25 KGy (Kilo Grays). This is a level of treatment that will render the sample essentially sterile with respect to viable microorganisms. The samples were left in the sealed zip lock bags for the treatment and were not opened until returned back to the laboratory.

In physical appearance, the pectin was not noticeably different than the untreated sample. It did not show signs of darkening, there were no unusual odors in the sample and it was possible to dissolve the sample in water very easily. A solution over 13% (weight / weight basis) could be prepared quite easily.

The following formulation was used to test the effectiveness of the gamma treatment. This pectin was treated with a radiation level of 25 KGy from a cobalt source.

| Ingredient | Weight | % |
|---|---|---|
| Water - Deionized | 180.00 | 82.15 |
| Pectin | 30.00 | 13.69 |
| Flavor (peppermint oil) IFF | 5.00 | 2.28 |
| Glycerol | 2.30 | 1.05 |
| Polyethylene Glycol (PEG) 300 | 1.00 | 0.46 |
| Aspartame (Nutrasweet) | 0.70 | 0.40 |
| Blue #1 FD&C (1% Solution) | 0.09 | 0.04, |
| Yellow #5 FD&C (1% Solution) | 0.02 | 0.01 |
| Total | 219.11 | 100.00 |

Films were cast using a draw down bar on plastic sheet. The initial film thickness was about 0.0075 inches and the final thickness of the film was 0.002 to 0,0015. This is typical of the breath strips currently on the market in the United States.

The product made with the gamma treated pectin clearer, slightly more brittle, and smoother and glossier in appearance than a product made using pH treatment. However, to achieve the same level of flexibility in the film required using about double the glycerol level in the gamma treated product than in the pH treated product. With this increase in plasticizer, the film could be bent double (hairpin shape) and it would crease but not break.

Example 2

This example is made with pectin treated at 15 KGy using a cobalt source. It was found to dissolve slightly slower than the film described in Example 1.

| Ingredient | Weight (g) | % |
|---|---|---|
| Water-Deionized | 180 | 82.907 |
| Pectin | 28 | 12.897 |
| Flavor (peppermint oil) IFF | 5 | 2.303 |
| Glycerol | 2.3 | 1.059 |
| Polyethylene Glycol (PEG) 300 | 1 | 0.461 |
| Aspartame (Nutrasweet) | 0.7 | 0.322 |
| Blue #1 FD&C (1% Solution) | 0.09 | 0.041 |
| Yellow #5 FD&C (1% Solution) | 0.02 | 0.009 |
| Total | 217.11 | 100.000 |

Samples were prepared at 4% pectin level using a propeller mixer at 800 rpm in water that had sodium bicarbonate (a buffer) and sodium hexametphospate (a calcium sequestering agent) added at 0.1% and 0.2% respectively. This solution mixed for 2 hours and then was adjusted to 25 °C. Viscosity was measured with a Brookfield LVT # 3 spindle at 60 rpm. This table shows how the radiation dosage affects the viscosity of the pectin solution. Viscosity is an excellent indicator of molecular weight under these conditions.

| Irradiation Measured | Dose Viscosity |
|---|---|
| 0 | 1260.0 |
| 9.8 | 388 |
| 11,6 | 334 |
| 13.2 | 292 |
| 15 | 240 |
| 18.6 | 184 |
| 21.2 | 166 |

Gamma irradiation treatment is significantly easier to perform than the previously disclosed technologies since it does not require dissolving the pectin to treat it. Furthermore, the gamma irradiation treatment renders the product essentially sterile. This is an important characteristic for drugs and cosmetics. The dosage of 15 KGy is close to optimal and closely matches the product made with pH neutralization to pH 6.5. From the solution viscosity observed the material treated with 10 KGy is slightly lower than the pH treated product. The film made with gamma treated pectin was very quick to dissolve in the mouth and did not have as much gummy or sticky feeling than the pH treated control. Both products were superior to the untreated pectin with respect to film dissolution and lack of a sticky or tacky mouthfeel.

Example 3

A pectin mask film is prepared and applied to the face by first misting the skin with water, then applying the mask film and misting on a bit more water to adhere it to the skin. The water holding properties of pectin are used to help moisturize the skin and create a silky feeling to the skin.

| **INGREDIENTS** | **Grams** | **Percent** |
|---|---|---|
| Water, deionized | 347.8 | 69.6 |
| GENU_{®} pectin type X-939-04 | 43.5 | 8.7 |
| Glycerol | 65.2 | 13.0 |
| Silica | 43.5 | 8.7 |
| Fragrances to suit | | |
| Color (1% solution) to suit | | |
| | 500.0 | 100.0 |

Weigh out the glycerol and add to the water. Disperse the silica in the water/glycerol blend using good agitation. When the silica is fully dispersed, add the pectin while mixing at 1200 rpm by slowly sifting the pectin onto the vortex of the water. As the viscosity increases, increase the agitation such that the whole solution continues to mix. Mix for a minimum of 15 minutes. Remove excess air such as by centrifugation or vacuum. Pour a portion of the solution on to a plastic sheet (e.g. polystyrene) and using the draw down bar, draw the solution down into a thin even layers. Preferably, the films should be cast to form a final dried film of about 40 µm (1.5 mils). A typical starting point is 0.36 mm (14 mils). Dry the film to a water activity of 0.5 or for about 4 hours at 45°C. Cut as appropriate and package.

Example 4

A specialty make up film is prepared with pectin and glitter. This film can be readily dissolved in water creating a glitter dispersion.

| **INGREDIENTS** | **Grams** | **Percent** |
|---|---|---|
| Water, deionized | 395.0 | 79.0 |
| GENU_{®} pectin type X-939-04 | 63.2 | 12.6 |
| Glycerol | 16.8 | 3.36 |
| Glitter Flakes | 25.0 | 5.0 |
| | 500.0 | 100.0 |

Weigh out the glycerol and add to the water. Weigh out the pectin and while mixing at 1200 rpm, slowly sift the pectin onto the vortex of the water. As the viscosity increases, increase the mixing speed so that the entire solution continues to mix. Mix for a minimum of 15 minutes. Add the glitter following hydration of the pectin. Remove excess air such as by centrifugation or vacuum. Pour a portion of the solution on to a plastic sheet (e.g. polystyrene) and using the draw down bar, draw the solution down into a thin even layers. Preferably, the films should be cast to form a final dried film of about 40 µm (1.5 mils). A typical starting point is 0.36 mm (14 mils). Dry the film to a water activity of 0.5 or for about 4 hours at 45°C. Cut as appropriate and package.

Example 5

A film containing an acne inhibitor is prepared and used to treat and conceal blemishes. The film is applied to moistened skin with a dry finger and rub gently to blend into the skin. Pectin helps to sooth the skin and the salicylic acid helps to heal the blemish.

| **INGREDIENTS** | **Grams** | **Percent** |
|---|---|---|
| Water, deionized | 416.7 | 83.3 |
| GENU_{®} pectin type X-939-04 | 62.5 | 12.5 |
| Color | 15.0 | 3.0 |
| Glycerol | 5.0 | 1.0 |
| Salicylic acid* | 0.8 | 0.2 |
| | 500.0 | 100.0 |

| | | |
|---|---|---|
| *concentration in dried film is about 1%. | | |

Weigh out the glycerol and add to the water in the 1000 ml beaker. Weigh out the pectin and while mixing at 1200 rpm, slowly sift the pectin onto the vortex of the water. As the viscosity increases, increase the mixing speed so that the entire solution continues to mix. Mix for a minimum of 15 minutes. Add the color following hydration of the pectin. Add the salicylic acid. Remove excess air such as by centrifugation or vacuum. Pour a portion of the solution on to a plastic sheet (e.g. polystyrene) and using the draw down bar, draw the solution down into a thin even layers. Preferably, the films should be cast to form a final dried film of about 40 µm (1.5 mils). A typical starting point is 0.36 mm (14 mils). Dry the film to a water activity of 0.5 or for about 4 hours at 45°C. Cut as appropriate and package.

Example 6

A film is prepared which can be cut into various shapes and applied to moistened skin. The film will readily stick to the skin but can be easily washed off. The pectin makes it safe and non irritating to the skin.

| **INGREDIENTS** | **Grams** | **Percent** |
|---|---|---|
| Water, deionized | 427.8 | 85.6 |
| GENU_{®} pectin type X-939-04 | 62.5 | 12.5 |
| Glycerol | 7.2 | 1.4 |
| Titanium dioxide | 2.5 | 0.5 |
| Color (pigment food grade) to suit | | |
| | 500.0 | 100.0 |

Weigh out the glycerol and add to the water in the 1000 ml beaker. Weigh out the pectin and while mixing at 1200 rpm, slowly sift the pectin onto the vortex of the water. As the viscosity increases, increase the mixing speed so that the entire solution continues to mix. Mix for a minimum of 15 minutes. The pigment and titanium dioxide which have been thoroughly blended (motor and pestle) should be added following the hydration of the pectin. (Note: glitter up to 5% can also be added for effect.) Remove excess air such as by centrifugation or vacuum. Pour a portion of the solution on to a plastic sheet (e.g. polystyrene) and using the draw down bar, draw the solution down into a thin even layers. Preferably, the films should be cast to form a final dried film of about 40 µm (1.5 mils). A typical starting point is 0.36 mm (14 mils). Dry the film to a water activity of 0.5 or for about 4 hours at 45°C. Cut as appropriate and package.

Example 7

A film is prepared which can be applied to sun sensitive areas of the skin. The film is easily applied to moistened skin and can be easily washed off after use.

| **INGREDIENTS** | **Grams** | **Percent** |
|---|---|---|
| Water, deionized | 417.5 | 83.5 |
| GENU_{®} pectin type X-939-04 | 62.5 | 12.5 |
| Titanium dioxide | 15.0 | 3.0 |
| Glycerol | 5.0 | 1.0 |
| | 500.0 | 100.0 |

Weigh out the glycerol and add to the water in the 1000 ml beaker. Weigh out the pectin and while mixing at 1200 rpm, slowly sift the pectin onto the vortex of the water. As the viscosity increases, increase the mixing speed so that the entire solution continues to mix. Mix for a minimum of 15 minutes. After the pectin has hydrated, add the titanium dioxide. Remove excess air such as by centrifugation or vacuum. Pour a portion of the solution on to a plastic sheet (e.g. polystyrene) and using the draw down bar, draw the solution down into a thin even layers. Preferably, the films should be cast to form a final dried film of about 40 µm (1.5 mils). A typical starting point is 0.36 mm (14 mils). Dry the film to a water activity of 0.5 or for about 4 hours at 45°C. Cut as appropriate and package.

Example 8

A pectin and xanthan gum film can be used to deliver hair color in precise locations. Various colors can be used. The hair should be dampened then the film applied with dry hands. Mist the hair and film with water to dissolve the film and release the color. A quick initial release of color is achieved. The xanthan will delay the complete dissolution of the film and allow for longer penetration times.

| **INGREDIENTS** | **Grams** | **Percent** |
|---|---|---|
| Water, deionized | 439.9 | 88.0 |
| GENU_{®} pectin type X-939-04 | 38.3 | 7.7 |
| KELTROL_{®} xanthan gum | 5.0 | 1.0 |
| Glycerol | 14.3 | 2.9 |
| Color (1% solution) to suit | 2.6 | 0.5 |
| | 500.0 | 100.0 |

Weigh out the glycerol and add to the water in the 1000 ml beaker. Weigh out the pectin and xanthan gum and, while mixing at 1200 rpm, slowly sift the gums onto the vortex of the water. As the viscosity increases, increase the mixing speed so that the entire solution continues to mix. Mix for a minimum of 15 minutes. Remove excess air such as by centrifugation or vacuum. Pour a portion of the solution on to a plastic sheet (e.g. polystyrene) and using the draw down bar, draw the solution down into a thin even layers. Preferably, the films should be cast to form a final dried film of about 40 µm (1.5 mils). A typical starting point is 0.36 mm (14 mils). Dry the film to a water activity of 0.5 or for about 4 hours at 45°C. Cut as appropriate and package.

Suitable pectin films within the invention may be obtained by substituting the generically and specifically described constituents and/or operating conditions of this invention for those used in the preceding examples.

## Claims

1. A process for making a rapidly dissolving pectin film, comprising the steps of: irradiating pectin with gamma radiation; and forming a film from the gamma radiation treated pectin.

2. The process of claim 1 further comprising irradiating dry pectin with gamma radiation, forming a solution with the gamma radiation treated pectin, and forming a film from the solution.

3. The process of claim 1 further comprising forming a solution from the pectin, irradiating the pectin solution with gamma radiation, and forming a film from the solution.

4. The process of claim 1 wherein the gamma radiation is produced from an electron beam or a cobalt source.

5. The process of claim 1 wherein the gamma radiation dosage is from about 10 to about 30 KGy,

6. The process of claim 5 wherein the gamma radiation dosage is from about 10 to about 20 KGy.

7. The process of claim 1 further comprising combining the pectin with an active ingredient or a flavoring aid.

8. The process of claim 1 further comprising combining the pectin with a plasticizer.

9. A rapidly dissolving pectin film comprising pectin treated with gamma radiation.

10. The pectin film of claim 9 further comprising at least one active agent.

11. The film of claim 9 wherein the pectin is treated with gamma radiation at a dosage of from about 10 to about 30 KGy,

12. The film of claim 11 wherein the dosage is from about 10 to about 20 KGy.

13. The film of claim 9 further comprising an effective amount of a plasticizer.

14. The film of claim 9 wherein the pectin is a high methyl ester pectin.

15. The film of claim 9 wherein the pectin is a low methyl ester pectin.

16. The film of claim 9 wherein the pectin is present at a concentration of about 20 to about 80% by weight based on the dry weight of the film.

17. The film of claim 9 further comprising a flavoring aid.

18. The film of claim 10 wherein the active agent is a pharmaceutical.

19. The film of claim 10 wherein the active agent is menthol, benzocaine, or mixtures thereof.

20. A composition for producing a film which is rapidly dissolvable in water, comprising pectin treated with gamma radiation.

21. The composition of claim 20 further comprising at least one active agent or flavoring aid.

22. The composition of claim 20 wherein the pectin is treated with gamma radiation at a dosage of from about 10 to about 30 KGy,

23. The composition of claim 22 wherein the dosage is from about 10 to about 20 KGy.

## Patentansprüche

1. Verfahren zur Herstellung eines schnell auflösenden Pektinfilms, umfassend die Schritte: Bestrahlen des Pektins mit Gammastrahlung; und Ausbilden eines Films aus dem mit Gammastrahlung behandelten Pektin.

2. Verfahren nach Anspruch 1, ferner umfassend das Bestrahlen trockenen Pektins mit Gammastrahlung, Ausbilden einer Lösung mit dem mit Gammastrahlung behandelten Pektin und Ausbilden eines Films aus der Lösung.

3. Verfahren nach Anspruch 1, ferner umfassend das Ausbilden einer Lösung aus dem Pektin, Bestrahlen der Pektinlösung mit Gammastrahlung und Ausbilden eines Films aus der Lösung.

4. Verfahren nach Anspruch 1, wobei die Gammastrahlung aus einem Elektronenstrahl oder einer Kobaltquelle produziert ist.

5. Verfahren nach Anspruch 1, wobei die Gammastrahlungsdosierung von ungefähr 10 bis ungefähr 30 KGy beträgt.

6. Verfahren nach Anspruch 5, wobei die Gammastrahlungsdosierung von ungefähr 10 bis ungefähr 20 KGy beträgt.

7. Verfahren nach Anspruch 1, ferner umfassend das Kombinieren des Pektins mit einer Wirksubstanz oder einem Aromastoffhilfsmittel.

8. Verfahren nach Anspruch 1, ferner umfassend das Kombinieren des Pektins mit einem Weichmacher.

9. Schnell auflösender Pektinfilm, umfassend Pektin, das mit Gammastrahlung behandelt wurde.

10. Pektinfilm nach Anspruch 9, ferner umfassend mindestens einen Wirkstoff.

11. Film nach Anspruch 9, wobei das Pektin mit Gammastrahlung bei einer Dosierung von ungefähr 10 bis ungefähr 30 KGy behandelt ist.

12. Film nach Anspruch 11, wobei die Dosierung von ungefähr 10 bis ungefähr 20 KGy beträgt.

13. Film nach Anspruch 9, ferner umfassend eine wirksame Menge an einem Weichmacher.

14. Film nach Anspruch 9, wobei das Pektin ein hohes Methylester-Pektin ist.

15. Film nach Anspruch 9, wobei das Pektin ein niedriges Methylester-Pektin ist.

16. Film nach Anspruch 9, wobei das Pektin in einer Konzentration von ungefähr 20 bis ungefähr 80 Gew.%, basierend auf dem Trockengewicht des Films, vorliegt.

17. Film nach Anspruch 9, ferner umfassend ein Aromastoffhilfsmittel.

18. Film nach Anspruch 10, wobei der Wirkstoff ein Arzneimittel ist.

19. Film nach Anspruch 10, wobei der Wirkstoff Menthol, Benzocain oder Mischungen aus diesen ist.

20. Zusammensetzung zur Produzierung eines Films, der schnell in Wasser auflösbar ist, umfassend Pektin, das mit Gammastrahlung behandelt ist.

21. Zusammensetzung nach Anspruch 20, ferner umfassend mindestens einen Wirkstoff oder ein Aromastoffhilfsmittel.

22. Zusammensetzung nach Anspruch 20, wobei das Pektin mit Gammastrahlung bei einer Dosierung von ungefähr 10 bis ungefähr 30 KGy behandelt ist.

23. Zusammensetzung nach Anspruch 22, wobei die Dosierung von ungefähr 10 bis ungefähr 20 KGy beträgt.

## Revendications

1. Procédé permettant de fabriquer un film de pectine se dissolvant rapidement, comportant les étapes consistant à : irradier la pectine avec un rayonnement gamma; et former un film à partir de la pectine traitée aux rayons gamma.

2. Procédé selon la revendication 1 comprenant, de plus, l'irradiation d'une pectine sèche avec un rayonnement gamma, la formation d'une solution avec une pectine traitée aux rayons gamma et la formation d'un film à partir de la solution.

3. Procédé selon la revendication 1 comprenant, de plus, la formation d'une solution à partir de la pectine, l'irradiation de la solution de pectine avec un rayonnement gamma et la formation d'un film à partir de la solution.

4. Procédé selon la revendication 1 dans lequel le rayonnement gamma est produit à partir d'un faisceau d'électrons ou d'une source de rayonnement au cobalt.

5. Procédé selon la revendication 1 dans lequel le dosage du rayonnement gamma va d'environ 10 KGy à environ 30 KGy.

6. Procédé selon la revendication 5 dans lequel le dosage du rayonnement gamma va de 10 KGy environ à 20 KGy environ.

7. Procédé selon la revendication 1 comprenant, de plus, la combinaison de la pectine avec une substance active ou un adjuvant de matière odoriférante.

8. Procédé selon la revendication 1 comprenant, de plus, la combinaison de la pectine avec un agent plastifiant.

9. Film de pectine se dissolvant rapidement comportant de la pectine traitée avec un rayonnement gamma.

10. Film de pectine selon la revendication 9 comportant, de plus, au moins un agent actif.

11. Film selon la revendication 9 dans lequel la pectine est traitée avec un rayonnement gamma suivant un dosage allant de 10 KGy environ à 30 KGy environ.

12. Film selon la revendication 11 dans lequel le dosage s'effectue de 10 KGy environ à 20 KGy environ.

13. Film selon la revendication 9 comprenant, de plus, une quantité efficace d'un agent plastifiant.

14. Film selon la revendication 9 dans lequel la pectine est une pectine riche en ester méthylique.

15. Film selon la revendication 9 dans lequel la pectine est une pectine pauvre en ester méthylique.

16. Film selon la revendication 9 dans lequel la pectine est présente à une concentration de 20 % environ à 80% environ en poids sur la base du poids à sec du film.

17. Film selon la revendication 9 comprenant, de plus, un adjuvant de matière odoriférante.

18. Film selon la revendication 10 dans lequel l'agent actif est un produit pharmaceutique.

19. Film selon la revendication 10 dans lequel l'agent actif est du menthol, de la benzocaïne ou leurs mélanges.

20. Composition permettant de produire un film qui peut se dissoudre rapidement dans l'eau comprenant une pectine traitée avec un rayonnement gamma.

21. Composition selon la revendication 20 comprenant, de plus, au moins un agent actif ou un adjuvant de matière odoriférante.

22. Composition selon la revendication 20 dans laquelle la pectine est traitée avec un rayonnement gamma suivant un dosage allant de 10 KGy environ à 30 KGy environ.

23. Composition selon la revendication 22 dans laquelle le dosage va de 10 KGy environ à 20 KGy environ.
